# EUROPEAN PATENT SPECIFICATION

(11) **EP 4 054 614 B1**
(45) Date of publication and mention of the grant of the patent: **22.04.2026**
(21) Application number: 20800203.0
(22) Date of filing: 06.11.2020
(51) Int. Cl.: A61K 36/49, A61P 31/12

(54) **ANTIVIRAL PREPARATION FROM CHESTNUT SHELLS OF CASTANEA SATIVA MILL**
ANTIVIRALE ZUBEREITUNG AUS KASTANIENSCHALEN VON CASTANEA SATIVA MILL
PRÉPARATION ANTIVIRALE OBTENUE À PARTIR DE COQUES DE CASTANEA SATIVA MILL

(30) Priority: 06.11.2019 IT 201900020512
(43) Date of publication of application: 14.09.2022
(73) Proprietor: MICRONATURE S.R.L., 80138 Napoli (NA) (IT); La Cara, Francesco, 81100 Caserta (CE) (IT); Morana, Alessandra, 80131 Napoli (NA) (IT); Squillaci, Giuseppe, 80010 Quarto (NA) (IT)
(72) Inventor: FRANCI, Gianluigi, 80138 Napoli (NA) (IT); GALDIERO, Massimiliano, 80138 Napoli (NA) (IT); LA CARA, Francesco, 80138 Napoli (NA) (IT); MORANA, Alessandra, 80138 Napoli (NA) (IT); SQUILLACI, Giuseppe, 80138 Napoli (NA) (IT)
(74) Representative: Pace Napoleone, Maria
(86) International application number: PCT/EP2020/081329
(87) International publication number: WO 2021/089807

(56) References cited:
- WO-A1-2006/030567
- WO-A1-2006/030929
- CN-A- 104 069 105
- KR-A- 20040 064 425
- US-A1- 2010 040 713
- VELLA FILOMENA MONICA ET AL: "Valorization of the agro-forestry wastes from Italian chestnut cultivars for the recovery of bioactive compounds", EUROPEAN FOOD RESEARCH AND TECHNOLOGY, SPRINGER BERLIN HEIDELBERG, BERLIN/HEIDELBERG, vol. 245, no. 12, 29 October 2019 (2019-10-29), pages 2679 - 2686, XP036930887, ISSN: 1438-2377, [retrieved on 20191029], DOI: 10.1007/S00217-019-03379-W
- SQUILLACI GIUSEPPE ET AL: "Chestnut (Castanea sativaMill.) industrial wastes as a valued bioresource for the production of active ingredients", PROCESS BIOCHEMISTRY, vol. 64, 21 September 2017 (2017-09-21), pages 228 - 236, XP085326660, ISSN: 1359-5113, DOI: 10.1016/J.PROCBIO.2017.09.017
- LUPINI C ET AL: "In vitro antiviral activity of chestnut and quebracho woods extracts against avian reovirus and metapneumovirus", RESEARCH IN VETERINARY SCIENCE, BRITISH VETERINARY ASSOCIATION, LONDON, GB, vol. 87, no. 3, 1 December 2009 (2009-12-01), pages 482 - 487, XP026714187, ISSN: 0034-5288, [retrieved on 20090510], DOI: 10.1016/J.RVSC.2009.04.007
- WANG H X ET AL: "Purification of castamollin, a novel antifungal protein from Chinese chestnuts", PROTEIN EXPRESSION AND PURIFICATION, ACADEMIC PRESS, SAN DIEGO, CA, vol. 32, no. 1, 1 November 2003 (2003-11-01), pages 44 - 51, XP004469383, ISSN: 1046-5928, DOI: 10.1016/S1046-5928(03)00212-2

## Description

### SECTOR OF THE INVENTION

The present invention concerns the use of a natural extract and/or its fractions obtained from a waste produced through the industrial peeling processing of chestnuts (fruit from *Castanea sativa* Mill.). This extract and/or its fractions are used in pharmaceutical preparations and/or medical devices for the treatment of pandemic virus-mediated infections caused by viruses belonging to *Herpesviridae, Coronaviridae* and *Retroviridae* families.

### BACKGROUND ART

Nowadays, the major public health problems are microbial infections. Pandemic, resistant strains and new emerging microorganisms highlight the need to find efficacy, low toxic and ready to use approach and therapy to face the global problem. Some of those problems are caused by the increased resistance of pathogens to therapies. Over the past few decades, a number of viruses have first come to light or have reappeared, resulting in significant epidemics and pandemics. Today, several diseases due to viral infection are widespread globally. Among these groups for instance we can underline the presence of viruses such as Herpes Simplex Virus 1 and 2 (HSV-1, HSV-2), Severe Acute Respiratory Syndrome coronavirus 2 (SARS-CoV-2) and the Human Immunodeficiency Viruses (HIV). All these viruses share the global impact on the human population. HSV-1 and 2, the SARS-CoV-2, and HIV infected already millions of humans around the world. In addition, for some of these viruses there are efficient drugs for their treatment, see Acyclovir and derivatives for HSV-1 and 2, but the prolonged and intense use of them created resistant strains around the world.

### Herpes Simplex Virus (HSV)

Herpes Simplex Virus (HSV) infections represent one of the most common infectious diseases in humans. The virus can be classified into two types: herpes simplex type 1 (HSV-1) and type 2 (HSV-2) (Roizman et al. 2007). Both share the latency capacity but can be distinguished mainly based on the mode of transmission and the site where they establish the latency: HSV-1 is generally transmitted by oral contact, generating the so-called "cold sores" (herpes labialis), while HSV-2 causes genital herpes and sexually transmitted diseases (STDs). The latest WHO bulletin (https://www.who.int/news-room/fact-sheets/detail/herpes-simplex-virus) estimates that 3.7 billion people under the age of 50 (the 67% of the world population in 2012) are infected with HSV-1; in addition, the WHO noted that 417 million people aged from 15 to 49 (11%) have an HSV-2 infection.

Currently, various drugs such as acyclovir, penciclovir, famciclovir, valaciclovir and other guanine analogues are used for the treatment of HSV. However, even for herpes infections, the onset of drug resistance is common, to which is added the inability to eradicate latent infections (Cavalli et al. 2012).

### SARS-CoV-2

In early December 2019 in Wuhan, Hubei province in China, a new human infection was recorded. A new betacoronavirus disease was discovered and in a flash it reached a pandemic entity. In October 25, 2020, there is a staggering global number of 42,303,118 cases of COVID-19, including 1,145,695 deaths (https://covid19.who.int/?gclid=Cj0KCQjwxNT8BRD9ARIsAJ8S5xbHD0BMKsWyUvAm7sb x3veiugfdvbtHk_lw21KL_9LPzhdE0VwMyw4aAnYeEALw_wcB). The pandemic, brought about by the extreme intense respiratory condition infection (SARS-CoV-2), represents a serious danger to general wellbeing for the wide range of clinical appearances. Albeit about half of the tainted don't encounter any manifestations, the staying half can advance to pneumonia up to Acute Respiratory Distress Syndrome (ARDS) trailed by multi-organ brokenness condition (MODS) Multiple Organ Dysfunction Syndrome) and even passing (Zaim et al. 2020).

It is a positive-sense single-stranded RNA (+ssRNA) virus, with a single linear RNA segment. This virus belongs to the Coronaviridae Family, Betacoronavirus Genus and Sarbecovirus Subgenus. Other coronaviruses are capable of causing illnesses ranging from the common cold to more severe diseases such as Middle East respiratory syndrome (MERS, fatality rate ~34%). It is the seventh known coronavirus to infect people, after 229E, NL63, OC43, HKU1, MERS-CoV, and the original SARS-CoV (Zhu et al. 2020). Specific therapies and vaccines are under investigation

(https://clinicaltrials.gov/ct2/results?cond=Covid19&term=&cntry=&state=& city=& dist=). The clinical trial website clinicaltrials.gov reported more than 3700 studies of new drugs, repurposing drugs and vaccines ongoing for COVID-19 disease. At the moment no specific drugs are approved from the Food and Drug Administration (FDA).

### HIV

HIV is a member of the genus *Lentivirus* (International committee on taxonomy of viruses (2002), 61.0.6. Lentivirus, National institutes of health, Retrieved, February 28, 2006), part of the family Retroviridae (International Committee on Taxonomy of Viruses (2002). "61. Retroviridae". National Institutes of Health. Retrieved February 28, 2006). Lentiviruses have many morphologies and biological properties in common. Many species are infected by lentiviruses, which are characteristically responsible for long-duration illnesses with a long incubation period (Levy 1993). Lentiviruses are transmitted as single-stranded, positive-sense, enveloped RNA viruses. To date, more than 38 million people are HIV positive. In the last years the count of people that died of HIV infection was close to 1 million/year which peaked at 1,8 million in 2005 (http://www.healthdata.org/research-article/estimates-global-regional-and-national-incidence-prevalence-and-mortality-hiv-1980). Because a critical issue in the long-term clinical management of HIV disease is the development of drug resistance that often appears during HAART therapy, reducing its effectiveness, the inhibitory activity of ethyl acetate and acetone extracts from *Thymelaea hirsuta* (leaves and branches) were also tested against a panel of viruses possessing mutations that confer selective resistance either to nucleoside (NRTI) ornon-nucleoside (NNRTI) reverse transcriptase (RT) inhibitors. In this scenario, natural products have been the most successful source of inspiration for the development of new drugs.

One of the main problems in HIV eradication is the latency. The possibility to have a viral reservoir in CD4+ T cells, dendritic cells, as well as macrophages, remains the main barrier to virus treatment. Despite the fact that the HAART therapy is able to inhibit the HIV replication the HIV eradication is not possible with this approach. Indeed the HAART treatment is not able to kill the virus in the latency form. In this scenario, a combination of therapies among HAART and Histone Deacetylases Inhibitors (HDAC), also called "Kick and Kill" is able to force the HIV out from the latency phase and bring the HAART therapy working on them (https://onlinelibrary.wiley.com/doi/full/10.1038/icb.2011.95).

All together these infection diseases represent a major part of human infection and the possibility to treat them with a natural and common approach opens the window on a new perspective.

As a result, part of the scientific interest is now aimed at the research and development of new antimicrobials alternatives to current pharmacological products, possibly of natural origin; in particular, the attention is focused on extracts from plants as they contain mixtures of bioactive molecules which have been shown to be more active than the isolated forms due to their synergistic action (Dolatabadi et al. 2018; Pendota et al. 2018). The search for new sources for the production of active natural extracts is therefore constantly growing. According to this scenario, various antiviral activities from plant extracts, very often food plants, have been described in literature or have been patented in recent years.

As examples, patent application US2011027399 refers to the antiviral activity against non-enveloped viruses belonging to the genus *Betanodavirus, Aquavirnavirus, Ranavirus, Enterovirus, Mastadenovirus, Vesivirus,* and *Rotavirus* in extracts from *Diospyros kaki*; patent application US009364511 refers to the antiviral activity of an aqueous extract from cinnamon bark against enveloped viruses belonging to *Orthomyxovirus, Paramyxovirus, Herpesvirus, Retrovirus, Coronavirus, Hepadnavirus, Poxvirus, Togavirus, Flavivirus, Filovirus, Rhabdovirus,* and *Bunyavirus* genus.

A very interesting plant species, in this sense, is represented by *Castanea sativa* Mill. (European Chestnut). This tree represents an important species both for the contribution to human and animal nutrition, and for the economic value represented by the wood. *C. sativa,* due to its importance, has been extensively studied over the years in many researches aimed at identifying possible biotechnological applications that can be drawn from the plant itself (wood), from its fruits or from their waste (shells).

In most cases, these biotechnological activities are contained in extracts obtainable through simple, well-known and traditional procedures.

Patent Application KR20040064425 describes an inhibiting activity of the Cathepsin K enzyme. This enzyme is involved in the onset of osteoporosis. The inhibitory activity of the enzyme is contained in the chestnut extract obtained through a simple extraction in water.

Patent Application US20100040713 discloses the inhibiting activity on fat absorption by an extract from chestnut shells obtained with hydrophilic solvent.

Patent Applications WO2006/030567 and WO2006/030929 disclose an inhibitory activity towards alpha-amylase and alpha-glucosidase. This activity is present in extracts from *Fagaceae* (WO2006/030567A1) and more specifically in the chestnut shells of the *Fagacea Castanea sativa* (WO2006/030929A1). The active extracts are easily obtained using ethanol or aqueous ethanol. The extract therein discloses are therefore used as an active ingredient for delaying saccharide digestion or absorption.

Vella et al (2019), "Valorization of the agro-forestry wastes from Italian chestnut cultivars for the recovery of bioactive compounds*"*, refers to polyphenolic compounds of potential interest as antioxidants are extracted from various parts of the chestnut tree, including the residue of the peeling of the fruit (shells). The extraction process used for the shells is the traditional one, i.e. boiling in water followed by centrifugation and freeze-drying. The only activity therein identified consists in the antioxidant power of these extracts. No mention of possible antimicrobial or antiviral activities.

Squillaci et al. (2018), "Chestnut (Castanea sativa Mill.*) industrial wastes as a valued bioresource for the production of active ingredients*" refers to antioxidant, anti-inflammatory and moisturizing activities useful in the cosmetic industry obtained from chestnut shells, extracted by boiling. The water extraction process is the traditional one, i.e. a boiling in water followed by centrifugation and freeze-drying. No antiviral activity in the chestnut shells is therein disclosed.

Lupini et al. (2009), "In vitro antiviral activity of chestnut and quebracho woods extracts against avian reovirus and metapneumovirus*"* refers to extracts from chestnut and quebracho woods and their activities against avian *Reovirus* (ARV) and avian *Metapneumovirus* (AMPV).

According to the scientific literature and the existing patents, it is evident that many biotechnological activities of industrial interest are present in *Castanea sativa* and in chestnut shells, and many others could exist. It is also clear that these activities are obtained, in most cases, with simple and traditional extraction solvents, such as hot water or hydroalcoholic mixtures.

In the literature, some studies dealing with antiviral activities on human herpetic viruses and HIV extracted from plants other than *Castanea sativa* are reported (Fukuchi et al. 1989; Cheng et al. 2002; Helfer et al. 2014). Often, these plants are of food interest and their exploitation to obtain antiviral activities is in contrast with the current use. In this context, the exploitation of the large amounts of waste from anthropogenic activities, in particular agro-industrial residues, would represent a great resource, currently little considered, for the production of bioactive compounds (Lee et al. 2015; Abu-Lafi et al. 2017).

In 2018, 147,375 tons of chestnuts were produced and processed in the European Union and in this context, Italy is the first nation in terms of quantity produced, as it supplies about 36% of total production with 53,280 tons (http://www.fao.org/faostat/en/#data/QC). Through the peeling process, the chestnut processing industry produces large quantities of a solid residue that represents approximately 10-15% of the weight of the chestnut (Vázquez et al. 2009).

The most significant fraction of this residue (the shells) is made up of outer skin (pericarp) and inner skin (episperm), which are currently burned to solve the problem of their disposal. This treatment can create problems of environmental pollution since the combustion could generate toxic compounds similar to dioxin (Liberti et al. 1983). Chestnut shells represent an interesting source of bioactive molecules; in fact, they contain between 2.7 and 5.2% (w/w) of phenolic compounds, substances with several biological activities, including a marked antioxidant power (Jung et al. 2015; Ferri et al. 2016; Vázquez et al. 2008; De Vasconcelos et al. 2010). Therefore, it is still felt the need of a natural extract to be used as an alternative to synthetic drugs in the treatment of infections by *Herpesviridae, Retroviridae,* and *Coronaviridae* families.

KR20040064425 discloses a chestnut shell extract which inhibits the activity of the enzyme of Cathepsin K, and effectively prevents and treats osteoporosis together with a calcium preparation. US2010040713 discloses a fat absorption inhibitor containing a chestnut skin extract, and a food and drink containing that fat absorption inhibitor.

WO2006030567 discloses a plant-derived carbohydrase inhibitor, obtained by subjecting the shell, astringent skin, case, leaf, bark or seed (nut and cotyledon) of at least one plant selected from the group consisting of Castanea crenata, Quercus acutissxma, and Castanopsis cuspxdata to extraction using water, organic solvent, or a mixture thereof.WO2006030929 discloses a plant-derived carbohydrase inhibitor, wherein the inhibitor is effective for preventing or alleviating diabetes, or preventing obesity, and foods, drinks, and medicines containing the same, obtained by solvent extractions of astringent skins of chestnut.

Vella F.M. et al. Valorization of the agro-forestry wastes from Italian chestnut cultivars for the recovery of bioactive compounds. Eur Food Res Technol 245, 2679-2686 (2019). https://doi.org/10.1007/s00217-019-03379-w disclose biochemical characterization of chestnuts from four cultivars coming from the Italian Park "Roccamonfina-Foce Garigliano".

Squillaci G. et al., Chestnut (Castanea sativa Mill.) industrial wastes as a valued bioresource for the production of active ingredients, Process Biochemistry, 64, 2018, 228-236,https://doi.org/10.1016/j.procbio.2017.09.017. disclose by-products from chestnut peeling processing used for the production of active ingredients.

Lupini C, et al., In vitro antiviral activity of chestnut and quebracho woods extracts against avian reovirus and metapneumovirus. Res Vet Sci. 2009 Dec;87(3):482-7. doi: 10.1016/j.rvsc.2009.04.007. Epub 2009 May 10. disclose three different Chestnut (Castanea spp.) wood extracts and one Quebracho (Schinopsis spp.) wood extract, all containing tannins a tested for in vitro antiviral activity against avian reovirus (ARV) and avian metapneumovirus (AMPV). Wang HX, Ng TB. Purification of castamollin, a novel antifungal protein from Chinese chestnuts. Protein Expr Purif. 2003 Nov;32(1):44-51. doi: 10.1016/S1046-5928(03)00212-2 disclose an antifungal protein, designated castamollin, isolated from Chinese chestnut (Castanea mollisima). CN104069105 discloses THE application of castanapetmine in preparation of anti-AIDS drugs.

### SUMMARY OF THE INVENTION

The use of an antiviral extract discovered and obtained from an industrial waste, such as chestnut shells, represents an opportunity to enhance this waste and at the same time a new weapon in the fight against some viral infections. The authors thus analyzed the natural extracts obtained from chestnut shells (agro-industrial waste from chestnut peeling processing) of European chestnut (*Castanea sativa* Mill.) as a valid alternative to the treatment of viral infections by *Herpesviridae, Retroviridae,* and *Coronaviridae* families, reducing the risk deriving from the use of synthetic drugs.

The invention relates to the application of chestnut shells extract (CSE), or its fractions, or preparations where the active ingredient is represented by the CSE or fractions thereof, in the treatment and/or prevention of infections, caused by *Herpesviridae* and/or *Retroviridae* and/or *Coronaviridae.* These extracts have shown, regardless of the source of the shells (different suppliers and different peeling methodologies) and regardless the extraction method in *in vitro* experimental models, a very high efficacy in the treatment of infections caused by viruses such as HSV-1, HSV-2 (as model of *Herpesviridae* human infections), HIV (as model of *Retroviridae* human infections), and Sars-CoV-2 as model of *Coronaviridae* human infections.

With this invention the inventors also demonstrated that the antiviral activities present in plant extracts, and specifically in chestnut shells extracts, are not universal, but are directed towards specific viral types. Experiments conducted on BDVD virus (Bovine Viral Diarrhea Virus), belonging to the *Flaviviridae* family, in fact showed the ineffectiveness of the extract from chestnut shells in reducing the infectivity of this virus in an *in vitro* experimental model.

The invention claims the use of CSE and/or fractions thereof currently chemically characterized or not yet characterized, obtained by using traditional methods for the extraction of polyphenolic compounds from *Castanea sativa,* commonly used for other purposes (boiling water, maceration, extraction with solvents or other) and/or not traditional methods in the treatment or prevention of *Herpesviridae, Retroviridae,* and *Coronaviridae* infections.

The experimental results have shown that CSE, whatever their origin and regardless of the peeling method applied to the fruit, once dried and resuspended in water at a concentration of 40 mg/mL, completely inhibited the ability of infection, and therefore replication of HSV-1 and HSV-2 viruses starting from concentration of 2 micrograms (µg) of extract/mL of solution. This effect was also observed in HIV infection and replication, obtaining 50% inhibition with concentrations equal to 10 µg of extract/mL of solution. Moreover, coronavirus SARS-CoV-2 replication was evaluated in presence of CSE. Results highlight the capability to inhibit totally the SARS-CoV-2 growth with concentration higher than 20 µg of extract/mL, with a 50% of virus replication inhibition with a concentration of 6.05 µg/mL. Those results open the potential use of CSE for the treatment of SARS-CoV-2 on contaminated surfaces and for human treatment.

Finally, the toxicity data showed that CSE is not toxic even at concentrations 1,000 times higher than the curative dose (Curative dose 0.2 micrograms/milliliter - Non-toxicity at 2 milligrams/milliliter). CSE and/or fractions thereof can be included in galenic and cosmetic preparations and in medical devices in the form of gel, cream, soap, oil, lavender, lip gloss, etc.

### DETAILED DESCRIPTION OF THE INVENTION

The object of the invention is the use of an extract from chestnut shells (CSE), peeling waste of the chestnut, fruit of the tree *Castanea sativa* Mill. (European Chestnut), and/or fractions thereof in the treatment or prevention of *Herpesviridae, Retroviridae, and Coronaviridae* infections.

An object of the invention is therefore an extract from shells of chestnut (Chestnut Shell Extract or CSE) fruits of *Castanea sativa* Mill. and/or fractions thereof for use in the treatment and/or prevention of *Herpesviridae* and/or *Retroviridae* and/or *Coronaviridae* infections.

For *Herpesviridae* and/or *Retroviridae* and/or *Coronaviridae* infections it is intended viral infections caused by *Herpesviridae* and/or *Retroviridae* and/or *Coronaviridae.*

Preferably, the infection is an HSV-1, HSV-2, HIV or Sars-CoV-2 infection.

Preferably the chestnut shells are waste from chestnut fruits industrial peeling.

Preferably the extract or its fractions are obtained using traditional methods for the extraction of polyphenolic compounds from *Castanea sativa* (e.g. boiling water, maceration, extraction with solvents) and/or not traditional methods (e.g. microwaves, ultrasounds, supercritical extraction). Preferably the extract or its fractions are obtained from industrial chestnut shells waste as burned shells *(Brulage* method), dried shells or by manual chestnut peeling.

Another object of the invention is a pharmaceutical composition or supplement or medical device comprising an extract from shells of chestnut fruits (CSE) of *Castanea sativa* Mill. and/or fractions and at least one excipient and/or carrier, for use in the treatment and/or prevention of *Herpesviridae* and/or *Retroviridae* and/or *Coronaviridae* infections. Preferably the excipient and/or carrier is pharmaceutically acceptable. Preferably the infection is caused by a human virus. Preferably the *Herpesviridae* infection is caused by a Alphaherpesvirinae, more preferably by Herpes simplex virus 1 or 2 (HSV-1 or HSV-2) infection.

Preferably the *Retroviridae* infection is caused by a lentivirus, more preferably by Human immunodeficiency virus 1 or Human immunodeficiency virus 2 infection.

Preferably the *Coronaviridae* infections is caused by a Betacoronavirus, more preferably a Sarbecovirus, even more preferably Sars-CoV-2, MERS-CoV, 229E, NL63, OC43, HKU1 virus. Preferably the infection is an HSV-1, HSV-2, HIV or Sars-CoV-2 infection.

Preferably the excipient is selected from the group consisting of: emollient (e.g. glycerin and/or other), gelling agent (e.g. polyacrylamide, isoparaffin, laureth 7 and/or other), preservative (e.g. 2-phenoxyethanol, 3-(2-ethylhexyloxy)propane-1,2-diol and/or other), alcohol, surfactant, bactericide and solvent (e.g. aqueous solutions, buffered physiological solution and/or other) and combinations thereof.

Preferably the amount of the extract and/or of fractions thereof ranges from 0.000002% to 20% by weight of the total amount of the composition or supplement or device.

In a preferred embodiment the chestnut shells are waste from chestnut fruits industrial peeling.

Preferably the extract is obtained using traditional methods for the extraction of polyphenolic compounds from *Castanea sativa* (e.g. boiling water, maceration, extraction with solvents) and/or not traditional methods (e.g. microwaves, ultrasounds, supercritical extraction).

Preferably the extract is obtained from industrial chestnut shells waste as burned shells *(Brulage* method), dried shells or by manual chestnut peeling.

Preferably the virus is human.

Preferably the *Herpesviridae* virus is a Alphaherpesvirinae virus, more preferably Herpes simplex virus 1 and 2 (HSV-1 and HSV-2).

Preferably the *Retroviridae* virus is a lentivirus, more preferably Human immunodeficiency virus 1 or Human immunodeficiency virus 2.

In a preferred embodiment the virus is HSV-1, HSV-2, HIV or Sars-CoV-2.

Preferably the extract and/or fractions thereof ranges from 0.000002% to 20% by weight of the total amount of the product.

Preferably the chestnut shells are waste from chestnut fruits industrial peeling.

Preferably the extract or its fractions are obtained using traditional methods for the extraction of polyphenolic compounds from *Castanea sativa* (e.g. boiling water, maceration, extraction with solvents) and/or not traditional methods (e.g. microwaves, ultrasounds, supercritical extraction). Preferably the extract or its fractions are obtained from industrial chestnut shells waste as burned shells *(Brulage* method), dried shells or by manual chestnut peeling.

Preferably the *Herpesviridae* virus is a Alphaherpesvirinae virus, more preferably Herpes simplex virus 1 and 2 (HSV-1 and HSV-2).

Preferably the *Retroviridae* virus is a lentivirus, more preferably Human immunodeficiency virus 1 or Human immunodeficiency virus 2.

Preferably the *Coronaviridae* virus is a Betacoronavirus, more preferably a Sarbecovirus, even more preferably Sars-CoV-2, MERS-CoV, 229E, NL63, OC43, HKU1 virus.

Preferably the virus is HSV-1, HSV-2, HIV or Sars-CoV-2.

Preferably the extract and/or fractions thereof ranges from 0.000002% to 20% by weight of the total amount of the composition.

Preferably the chestnust shells are waste from chestnut fruits industrial peeling.

Preferably the extract or its fractions are obtained using traditional methods for the extraction of polyphenolic compounds from *Castanea sativa* (e.g. boiling water, maceration, extraction with solvents) and/or not traditional methods (e.g. microwaves, ultrasounds, supercritical extraction). Preferably the extract or its fractions are obtained from industrial chestnut shells waste as burned shells (*Brulage* method), dried shells or by manual chestnut peeling.

In the context of the present invention, "chestnut" and "chestnut fruit" are intended as interchangeably terms. The term *"Castanea sativa* Mill. chestnuts" may be used instead of "chestnut fruits of *Castanea sativa* Mill.".

The expressions "extract from shells of chestnut fruits (CSE) of *Castanea sativa* Mill." and *"Castanea sativa* Mill. chestnuts shells extract (CSE)" are interchangeable expressions.

In the context of the present invention, a "derivative" includes "extract". In the context of the present invention, chestnut shells also include chestnut peels.

The present invention also includes CSE of any plant belonging to the genus *Castanea,* preferably belonging to *Castanea Sativa,* more preferably to *Castanea sativa* Mill.

CSE is obtainable by means of already known extraction methods used for polyphenolic compounds. These methods include boiling water, maceration, extraction with solvents as examples.

CSE is obtainable from industrial chestnut shells produced through different peeling processes, as for example 1) burned shells, obtained by the *Brulage* method (chestnuts pass for a few seconds in a red-hot metal cylinder at a temperature of about 900 °C. The thermal shock to which chestnuts are subjected causes the shell to detach from the edible part), 2) dried shells, obtained by the following procedure: the drying process involves a treatment with hot air for 13-14 days, the time necessary for the chestnut to lose 50% of its water, and subsequently, the dried chestnuts are passed through a peeling machine, or 3) manual chestnut peeling.

The present extract (or fractions thereof) and compositions (or products or devices) comprising it inhibit proliferation of viruses belonging to the *Herpesviridae* and/or *Retroviridae* and/or *Coronaviridae* viral families.

Preferably, the present extract (or fractions thereof) and compositions (or products or devices) is specific and/or selective for viral types belonging to *Herpesviridae* and/or *Retroviridae* and/or *Coronaviridae* viral families.

The composition (or product or device) as defined above may further comprise at least one further antiviral agent.

The composition or product or device, according to the invention, can further comprise at least one of the following: solvents, stabilizers and excipients such as for example propylene glycol, polyethylene glycol, stearic acid and its salts of magnesium and calcium, gum arabic and xanthorrhea, fat emulsions, glycerin, vegetable oils or injectable organic esters, carbohydrates, such as glucose, sucrose, dextran, starch, maltodextrin, pullulans, silica, talc, antioxidants, ascorbic acid, glutathione, chelating agents , low molecular weight proteins.

Preferably the composition or product according to the invention further comprises glycerine, polyacrylamide, isoparaffin, laureth 7, 2-phenoxyethanol, 3-(2-ethylhexyloxy)propane-1,2-diol. Preferably it is in the form a cream.

In an embodiment of the invention, the composition for herpes treatment or against herpes can comprise in the formulation of a cream based on chestnut shells extract, the following excipients: glycerine, polyacrylamide, isoparaffin, laureth 7, 2-phenoxyethanol, 3-(2-ethylhexyloxy)propane-1,2-diol.

Preferably the composition according to the invention comprises:

| | |
|---|---|
| 1) Chestnut shells freeze-dried | 0,20% p/p |
| 2) Glycerine | 5,00% p/p |
| 3) Polyacrylamide | 1,40% p/p |
| 4) Isoparaffin | 0,70% p/p |
| 5) Laureth 7 | 0,19% p/p |
| 6) 2-phenoxyethanol | 0,10% p/p |
| 7) 3-(2-ethylhexyloxy)propane-1,2-diol | 0.10% p/p |
| 8) Purified water just enough | to100 g |

The composition or product or device, according to the invention, can be in a form suitable to be administered orally, intravaginally, rectally, parenteral, intravenous, intramuscular, subcutaneous, intraorbital, intraperitoneal or by passive or facilitated absorption through the skin and/or incorporated into liposomes, microspheres or other polymeric matrices. Preferably the CSE according to the invention is in solid or liquid form.

The present invention will now be illustrated with examples with reference to the following figures.
**Figure 1****.** Evaluation panel of the inhibition mediated by the extract of chestnut shells on HSV-1, HSV-2 and cell cycle interference models.
**Figure 2****.** Comparison of interference in HSV-1 mediated infection on Vero cells with extracts from Burned and Dried chestnut shells.
**Figure 3****.** Comparison of the inhibition in HSV-1 mediated infection on Vero cells with extracts from Burned and Dried chestnut shells, obtained through different extraction and treatment methods.

### EXAMPLE 1

### Materials and methods

### Used strains:

a) *S. aureus* ATCC 6538: Gram-positive, asporigenic, aerobic and facultative anaerobic bacteria, immobile and belonging to the *Micrococcaceae* family, included in the genus *Staphylococcus*;
b) *E. coli* ATCC 11229: Gram-negative, asporigenic, aerobic and facultative anaerobic bacteria, motile for peritric cilia belonging to the *Enterobacteriaceae* family, included in the genus *Escherichia.*
*c) Candida albicans* ATCC 90028: dimorphic saprophytic fungus, belonging to the *Saccharomycetes* family.

### Production of the chestnut shell extract (CSE):

The preparation of the CSE with a commonly used and published method is reported here as an example. The chestnut shells (external and internal), coming from the peeling process of Italian chestnuts by drying (Dried) or burning (Burned), were brought to a constant weight in the oven at 55 °C, then blended with a kitchen blender before being subjected to the extraction of bioactive molecules. The extraction was carried out at 5% (w/v) in deionized water, for 60 minutes, at 100 °C. To compensate for evaporation, every 20 minutes the volume of the mixture subjected to extraction was restored to the initial level (Squillaci et al. 2018).

The extract was then centrifuged at 4000 rpm, at 4 °C for 60 minutes (5810R, Eppendorf), the pellet removed, the supernatant frozen at -20 °C, and subsequently lyophilized.

### Antibacterial assays:

Antibacterial assays were performed using the plate microdilution method, according to the guidelines established by the National Committee on Clinical Laboratory Standards (NCCLS). To standardize the bacterial suspension for the antimicrobial assay, fresh colonies of each strain, previously obtained on Brein Heart Infusion agar, were inoculated in liquid Brein Heart Infusion and incubated at 37 °C over-night. The bacterial suspension was resuspended in fresh medium and further incubated at 37 °C until reaching the exponential growth phase (1x10⁸ CFU/mL). Serial dilutions were performed to determine the bacterial concentration required for the assay (1x10⁶ CFU/mL). The test was carried out in sterile 96-well plates in which the extract was diluted in Brain Heart Infusion for a final volume of 100 µl in order to obtain the required concentrations (200 µg/mL - 0.39 µg/mL). Similarly, dilutions were performed for ampicillin, used as a positive control. Subsequently, 50 µl of bacterial culture (5x10⁵ CFU/well) were inoculated into each well. The antimicrobial activity of the CSEs was evaluated after 20 hours of incubation at 37 °C, by measuring the absorbance at 600 nm, through the TECAN Sunrise microplate reader. The experiments were performed in duplicate and the percentage of inhibition was calculated with respect to the strains not treated with the substance (Franci et al. 2018).

### Antiviral assays:

Cell lines were purchased from American Type Culture Collection (ATCC). The absence of mycoplasma contamination was checked periodically by the Hoechst staining method. Cell lines supporting the multiplication of RNA viruses were the following: CD4+ human T-cells containing an integrated HTLV-1 genome (MT-4); Madin Darby Bovine Kidney (MDBK) [ATCC CCL 22 (NBL-1) Bos Taurus]; Baby Hamster Kidney (BHK-21) [ATCC CCL 10 (C-13) Mesocricetus auratus], Vero cells (CCL-81; American Type Culture Collection, Manassas, VA, USA)

Viruses were purchased from American Type Culture Collection (ATCC), with the exception of Human Immunodeficiency Virus type-1 (HIV-1 IIIB laboratory strain) obtained from the supernatant of the persistently infected H9/IIIB cells (NIH 1983); SARS-CoV-2 [Clinical isolate]; bovine viral diarrhoea virus (BVDV) [strain NADL (ATCC VR-534)]. The HSV-1 and HSV-2 viruses belong to the *Herpesviridae* family, the *Alphaherpesvirinae* subfamily, and the *Simplexvirus* genus. They express the gene for β-galactosidase, under the control of the cytomegalovirus IE-1 promoter and have been propagated as reported in the literature (Franci et al. 2017).

### Viral activity assays:

HSV-1 and HSV-2: To evaluate the effect of the CSE on the inhibition of HSV infectivity, a co-treatment experiment was performed, in which cells (4x10^{5/}multiwell 12 well) were incubated, simultaneously, with both CSE at different concentrations (200, 100, 50, 20, 2, 1, 0.5, 0.4, 0.3, 0.2, 0.1 and 0.05 µg/mL) and with the virus (10³ PFU/mL) for 1 hour at 37 °C/5% CO₂. After the incubation hour, time required to allow viral adsorption, the non-penetrated virus was inactivated with citrate buffer (pH 3.0). Subsequently, the cell monolayer was washed with 1X Phosphate Buffer Saline (PBS) and incubated for 48 hours in MEM supplemented with carboxymethylcellulose. After two days, the cells were fixed and stained with 0.5% crystal violet, and the plaques were counted. The experiments were performed in duplicate and the percentage of viral inhibition was calculated with respect to the HSV-1 and HSV-2 control not treated with the substance. We initially evaluated, by means of co-treatment experiments, the inhibitory capacity of this extract on HSV-1. The cells were incubated with different concentrations of the substance (200, 100, 50, 20, 2, 1, 0.5, 0.4, 0.3, 0.2, 0.1 and 0.05 µg/mL) in the presence of the virus (10³ PFU/mL) for 1 hour at 37 °C / 5% CO₂. After the incubation hour, time required to allow viral adsorption, the non-penetrated virus was inactivated with citrate buffer (pH 3.0).

Later, further experiments were carried out to understand whether CSEs acted in an intracellular or extracellular phase of infection. To assess whether they inhibited viral replication and therefore acted inside the cell, a post-treatment assay was set up: the cells were first infected with HSV-1 virus (10³ PFU/mL) for 1 hour at 37 °C / 5% CO₂. After one hour of incubation, the non-penetrated virus was inactivated with citrate buffer (pH 3.0) and the cell monolayer was washed with 1X Phosphate Buffer Saline (PBS). We continued by adding the extract to the cells at different concentrations (200, 20, 2 and 0.2 µg/mL) and incubating for a further hour at 37 °C / 5% CO₂ . To evaluate, instead, if the extracts acted in an extracellular phase, cell pre-treatment and virus pre-treatment experiments were carried out. Cell pre-treatment evaluates whether the substance acts on cells, for example by masking a binding receptor for the virus. In this experiment, the cells are first treated with substances at different concentrations (200, 20, 2 and 0.2 µg/mL) for 1 hour at 4 °C: this temperature allows the substance to interact with the cells, but not to enter them. After the incubation time, the cells are infected with the virus (10³ PFU/mL) and incubated for 1 hour at 37 °C / 5% CO₂. Virus pre-treatment, on the other hand, indicates whether CSEs act directly on the virus. In this experiment, the extract at different concentrations (200, 100, 50, 20, 2, 1, 0.5, 0.4, 0.3, 0.2, 0.1 and 0.05 µg/mL) is incubated together with the virus (10⁴ PFU/mL) for 1 hour at 37 °C. Subsequently the mixture is diluted and added on the cells for 1 hour at 37 °C/5% CO₂. For all experiments, at the end of the treatment, the cell monolayer was washed with 1X PBS and incubated for 48 hours in MEM supplemented with carboxymethylcellulose. After two days, the cells were fixed and stained with crystal violet and the plaques were counted. The percentage of viral inhibition was calculated with respect to the HSV control not treated with the substance.

HIV: CSE activity against HIV-1 was based on inhibition of virus-induced cytopathogenicity in MT-4 cell acutely infected with a multiplicity of infection (m.o.i.) of 0.01. Briefly, 50 µL of RPMI containing 1 x 10⁴ MT-4 cells were added to each well of flat-bottom microtitre trays, containing 50 µL of RPMI without or with serial dilutions of test compounds. Then, 20 µL of a HIV-1 suspension containing 100 CCID50 were added. After a 4-day incubation at 37 °C, cell viability was determined by the MTT method.

BVDV: CSE activity against BVDV was based on inhibition of virus-induced cytopathogenicity in BHK-21 and MDBK cells, respectively, acutely infected with a m.o.i. of 0.01. Briefly, BHK-21 and MDBK cells were seeded in 96-well plates at a density of 5 x 10⁴ and 3 x 10⁴ cells/well, respectively, and were allowed to form confluent monolayers by incubating overnight in growth medium at 37 °C in a humidified CO₂ (5 %) atmosphere. Cell monolayers were then infected with 50 µL of a proper virus dilution in maintenance medium [MEM-Earl with L-glutamine, 1mM sodium pyruvate and 0.025 g/L kanamycin, supplemented with 0.5 % inactivated FBS] to give an m.o.i of 0.01, without or with serial dilutions of test compounds. After a 3-day incubation at 37 °C, cell viability was determined by the MTT method (Pauwels et al. 1988). SARS-CoV-2 assays: SARS-CoV-2 field isolated was obtained from the University of Study of Campania Luigi Vanvitelli, Italy and propagated in Vero E6 cells. Virus stocks were generated from clarified cell culture supernatants harvested 3 or 4 days post inoculation. Virus titer and inhibition were determined by plaque assay in Vero E6 cells grown in six-well tissue culture plates (https://mbio.asm.org/content/11/5/e01935-20.long#sec-8).

### Cytotoxicity Assays

Cytotoxicity assays were run in parallel with antiviral assays.

Exponentially growing MT-4 cells were seeded at an initial density of 1 x 10⁵ cells/ml in 96-well plates in RPMI-1640 medium, supplemented with 10% fetal bovine serum (FBS), 100 units/mL penicillin G and 100 µg/mL streptomycin. Cell cultures were then incubated at 37 °C in a humidified, 5% CO₂ atmosphere, in the absence or presence of serial dilutions of test compounds. Cell viability was determined after 96 hrs at 37 °C by the 3-(4,5-dimethylthiazol-2-yl)-2,5-diphenyl-tetrazolium bromide (MTT) method (Pauwels et al. 1988).

MDBK cells were seeded in 96-well plates at an initial density of 6 x 10⁵ and 1 x 10⁶ cells/mL, respectively, in Minimum Essential Medium with Earle's salts (MEM-E), L-glutamine, 1mM sodium pyruvate and 25 mg/L kanamycin, supplemented with 10% horse serum (MDBK). Cell cultures were then incubated at 37 °C in a humidified, 5% CO₂ atmosphere in the absence or presence of serial dilutions of test compounds. Cell viability was determined after 72 hrs at 37 °C by the MTT method. The use of flow cytometry made it possible to evaluate the effect of CSE on the cell cycle. Vero cells (1 x 10⁶) were plated in 6-well multiwells and incubated the next day with the substance at the same concentrations used in the antiviral assays. After one hour, the cells were trypsinized and centrifuged, and the cell pellets were resuspended in a solution containing RNase A, propidium iodide (50 µg/mL), sodium citrate (0.1%) and Np40 (0.1%) in 1X PBS for 30 min in the dark. The analysis was performed at the FACScalibur flow cytometer (Becton Dickinson) as described previously (Franci et al. 2018).

### Antifungal assays:

The antifungal activity was performed using the plate microdilution method, according to the EUCAST guidelines. The test was carried out in sterile 96-well plates in which the CSE was diluted in the growth medium (RPMI-1640 with the addition of 2% glucose and 3- (N-morpholino)-propanesulphonic acid at the concentration of 0.165 mol/L) for a final volume of 200 µl in order to obtain the required concentrations (800 µg/mL - 0.39 µg/mL). Similarly, dilutions were performed for amphotericinB, used as a positive control. The fungal suspension used for the assay is prepared by resuspending colonies of *Candida albicans* (ATCC 90028), previously obtained from a 48 hour culture in Sabouraud Dextrose Agar medium, in sterile distilled water until reaching 0.5 McFarland. Serial dilutions were performed to determine the fungal concentration required for the assay (2.5x10⁵ CFU/mL). Subsequently, 100 µl of fungal culture (1.25x10⁵ CFU/well) was inoculated into each well. The antifungal activity of CSEs was evaluated after 48 hours of incubation at 37 °C, by measuring the absorbance at 600 nm, through the TECAN Sunrise microplate reader. The experiments were performed in duplicate and the percentage of inhibition was calculated with respect to the strains not treated with the substance (Cuenca-Estrella et al. 2003).

### Antitumor assays:

NB4 tumor line [DSMZ, ACC 207], acute promyelocytic leukemia, is propagated in RPMI 1640 medium containing 4.5 g/L supplemental glucose (Euroclone) with 10% bovine fetal serum (FBS) (Euroclone), 100 U/mL penicillin-streptomycin (Euroclone) and 2 mM L-glutamine (Euroclone). Cell cycle analysis: cells were plated at a confluence of 2 × 10⁵ cells/mL and induced for 30h with different concentrations of the substance (400, 200, 100, 50, 25, 12.5 and 0.78 µg/mL). They were then collected, centrifuged at 1200 rpm for 5 min and resuspended in a solution containing 1 × PBS, 0.1% sodium citrate, 0.1% NP40 and 50 mg/mL propidium iodide. After 30 min of incubation at room temperature, in the dark, the cell cycle is evaluated by FACS (FACSCalibur BD Biosciences, San Jose, CA, USA) and analyzed by ModFit v3 software (Verity Software House). The Pre-G1 phase was instead analyzed with CellQuestPro software (Becton Dickinson) (Lenoci et al. 2014; Franci et al. 2018).

### RESULTS

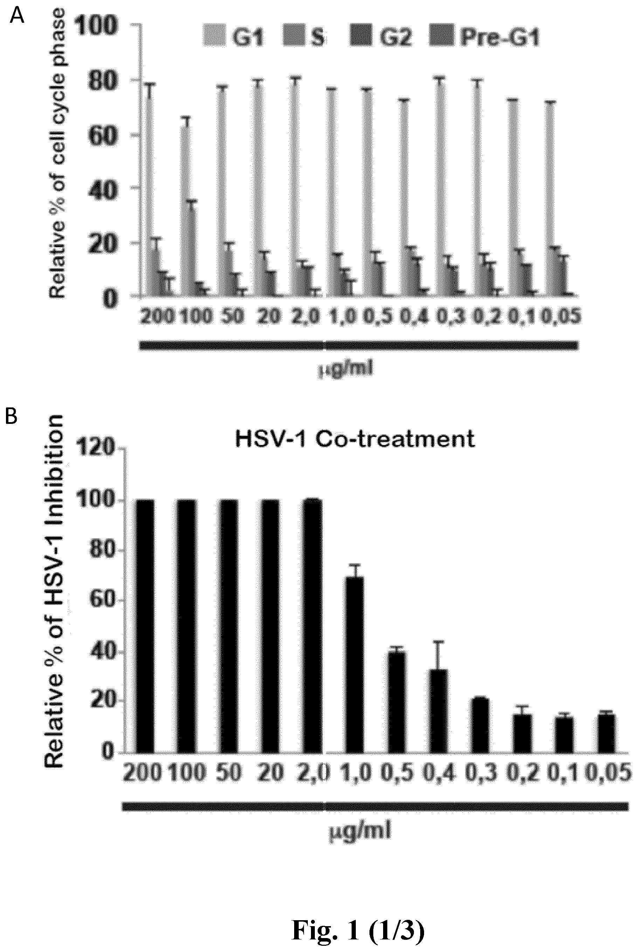

Before performing the cell tests, the right concentrations of CSE to be used were determined by analyzing cell cytotoxicity. Although a concentration curve was used to reach 200 µg/mL, we did not record changes in the cell cycle and in the increase in the Pre-G1 phase (death) of the cells treated for 24h with the extract on Vero Cells (Figure 1A). Other cell lines were tested for the toxic effect of CSE. Among them, MT-4 and MDBK were tested with a metabolic assay called MTT, values close to 100 µg/mL were still not toxic (See Tables 1-3). A complete panel of assays to evaluate the microbiological inhibitory activity of CSE was performed in biological duplicate and in technical triplicate. The results demonstrated that there was no interference with the growth of Gram- and Gram + bacteria even at the highest concentrations tested (200 µg/mL). Negative results were also acquired in response to *C*. *albicans* treatment (not showed). Unlike bacteria and fungi, treatment with CSE on viral models such as HSV-1, HSV-2, HIV-1, SARS-CoV-2 showed strong antiviral activity.

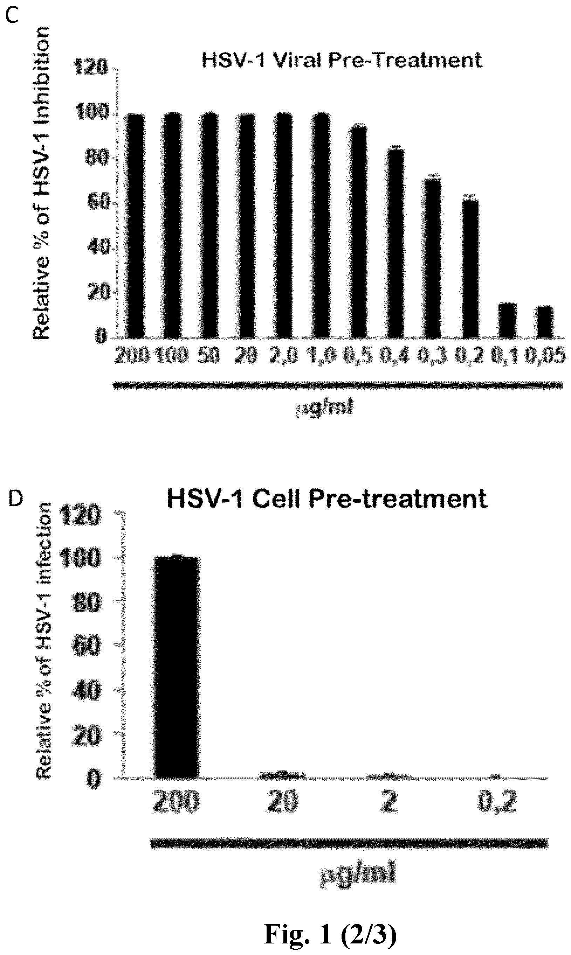

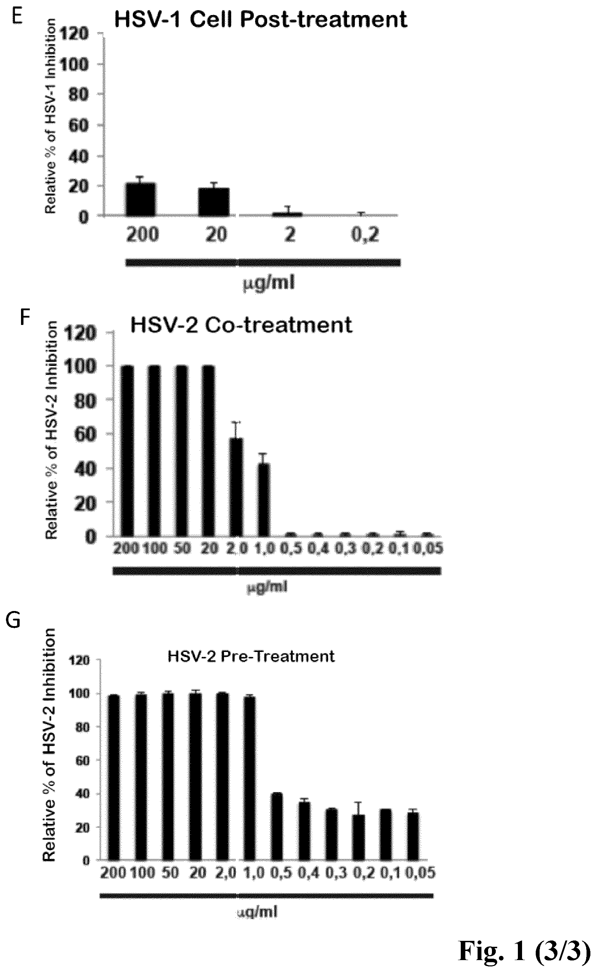

As can be seen from the experimental data obtained, the CSE shows a very strong inhibition of the infection of both HSV-1 and HSV-2 in the co-treatment experiments (Figure 1B and 1F) and an even more intense inhibition in the viral pre-treatment experiments (Figure 1C and 1G). The data indicate that in the cell pre-treatment the extract is no longer active, as well as in the post-treatment assay (Figure 1D and 1E). All the results obtained open a window on the mechanism of operation of CSE in the interference of viral infection: CSE works in an extracellular phase of HSV infection, and, more precisely, they act directly on the virus. We can hypothesize that the CSE interacts/binds a glycoprotein of the viral envelope, masking it from attack with its cell receptor and, therefore, preventing the attack on the target cell and its subsequent infection. The doses required to achieve 100% inhibition of viral replication are 2 µg/mL on HSV-1 in the co-treatment and 10 times less in the viral pre-treatment. For HSV-2, viral inhibition by CSE is reduced by 10 times: the concentrations required to block viral replication by 100% are 20 µg / mL in the co-treatment and 2 µg / mL in the virus pre-treatment. Such low doses and such high efficiency offer the possibility of using this extract in the treatment of HSV-1 and 2 infections with large margins of success.

To check whether the antiviral activity of CSE had a broader spectrum, further experiments were carried out on other virus models. CSE efficacy in infection mediated by the human immunodeficiency virus (HIV), responsible for acquired immunodeficiency syndrome (AIDS), was evaluated. It is a retrovirus of the lentivirus genus, which is divided into two strains: HIV-1 and HIV-2. The first of the two is mainly located in Europe, America and Central Africa; HIV-2, on the other hand, is found mostly in West Africa and Asia and results in a clinically more moderate syndrome than the previous strain. Antiviral treatments were conducted on H9/IIIB cells. The virus used (HIV-1 IIIB laboratory strain) was obtained from the supernatant of persistently infected H9/IIIB cells. The CSE was able to inhibit at least at 50% the virus replication at about 13.5 µg/mL. EFV and T20, two well known drugs able to inhibit HIV-1 replication were used as positive controls (Wang et al. 2011) (Table 1).

The CSE also inhibited at 50% the replication of SARS-CoV-2 at a value of 6.05 µg/mL. For these experiments, the peptide EK1 was used as control, as published in the paper by Xia et al. (2020) (Table 2).

No effect on replication of BVDV was achieved by CSE even at higher concentrations. For the BVDV trials, the reference drug NM108 was used as control (Tonelli et al. 2011) (Table 3).

It is really interesting how the CSE is able to inhibit some specific virus families and it is not active on all the viruses tested. This leaves the potential application of CSE in a specific virus category.

**Table 1: Cytotoxicity and in vitro activity of chestnut's shells extract against HIV-1.**

| **Extracts** | ***Cytotoxicity in MT-4 cells*** | ***In vitro efficacy EC₅₀^{b}[µg*/*mL]*** |
|---|---|---|
| | **CC₅₀^{a}[µg/mL]** | **HIV-1^{b}** |
| 40 mg/mL | 97.8 | 13 |
| 20 mg/mL | 96.0 | 14 |

| **Reference** | | |
|---|---|---|
| | CC₅₀^{a} | HIV-1^{b} |
| *EFV | 40.0 | 0.002 |
| T-20 (µg/ml) | >100 | 0.017 |

| | | |
|---|---|---|
| ^{a}Compound concentration (µg/mL) required to reduce the viability of mock-infected MT-4 cells by 50 %, as determined by the MTT method. ^{b}Compound concentration (µg/ml) required to achieve 50 % protection of MT-4 cells from the HIV-1 induced cytopathogenicity, as determined by the MTT method. *µM | | |

**Table 2: Cytotoxicity and in vitro activity of chestnut's extract against Coronaviridae.**

| **Extracts** | ***Cytotoxicity in VERO cells*** | ***In vitro efficacy EC₅₀^{b} [µg*/*mL]*** |
|---|---|---|
| | CC₅₀ ^{a} [µg/mL] | SARS-CoV-2^{b} |
| t 40 mg/mL | >500 | 6.2 |
| 20 mg/mL | >500 | 5.9 |

| **Reference** | | |
|---|---|---|
| | CC₅₀ ^{a} | SARS-CoV-2^{b} |
| ***EK1** | >100 | 5.4 |

| | | |
|---|---|---|
| ^{a}Compound concentration (µg/mL) required to reduce the viability of mock-infected MDBK cells by 50 %, as determined by the MTT method. ^{b}Compound concentration (µg/mL) required to achieve 50 % protection of VERO cells from SARS-CoV-2. *µM | | |

**Table 3: Cytotoxicity and in vitro activity of chestnut's extract against BVDV**

| **Extracts** | ***Cytotoxicity in MDBK cells*** | ***In vitro efficacy* EC₅₀ ^{b} [µg/mL]** |
|---|---|---|
| | CC₅₀ ^{a} [µg/mL] | BVDV^{b} |
| 40 mg/mL | 106.5 | >106.5 |
| 20 mg/mL | 109.0 | >109.0 |

| | | |
|---|---|---|
| **Reference** | CC₅₀ ^{a} | BVDV^{b} |
| ***NM 108** | >100 | 1.6 |

| | | |
|---|---|---|
| ^{a}Compound concentration (µg/mL) required to reduce the viability of mock-infected MDBK cells by 50 %, as determined by the MTT method. ^{b}Compound concentration (µg/mL) required to achieve 50 % protection of MDBK cells from BVDV. *µM | | |

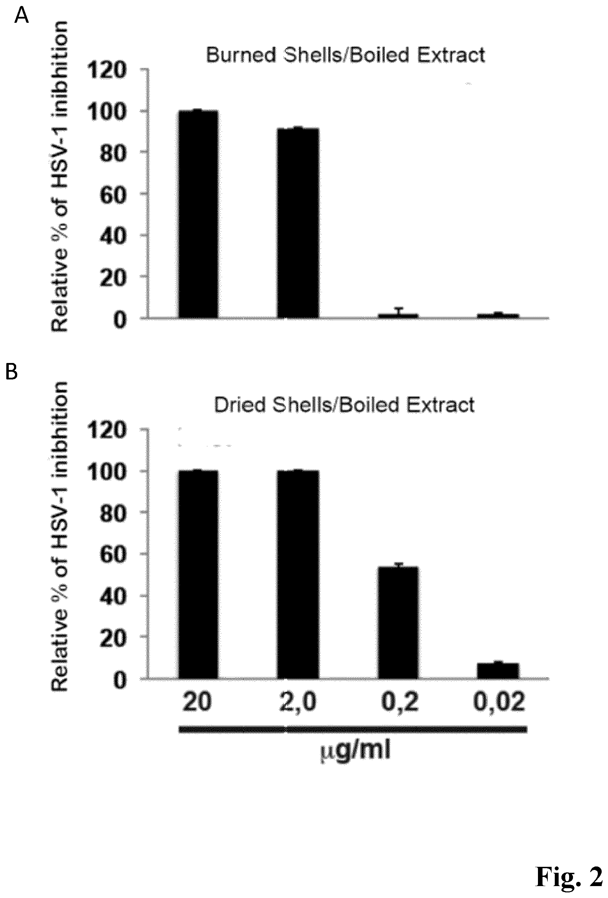

To confirm that the antiviral activity was really ascribed to CSE, regardless of the origin of the shells (obtained by drying, and called Dried, or by burning, and indicated as Burned), the effectiveness of the extracts from the two different types of shells was compared. Both extracts led to the same results, with an inhibition of HSV-1 infection, at a concentration of 2 µg/mL, close to 100% for the extract from Burned shells (Figure 2A) and equal to 100% for the extract from Dried shells (Figure 2B).

Furthermore, to confirm that the antiviral activity observed depended on the extract, regardless of the type of shells (Burned or Dried), and also of the type of extraction, CSEs produced with a method other than boiling were analyzed.

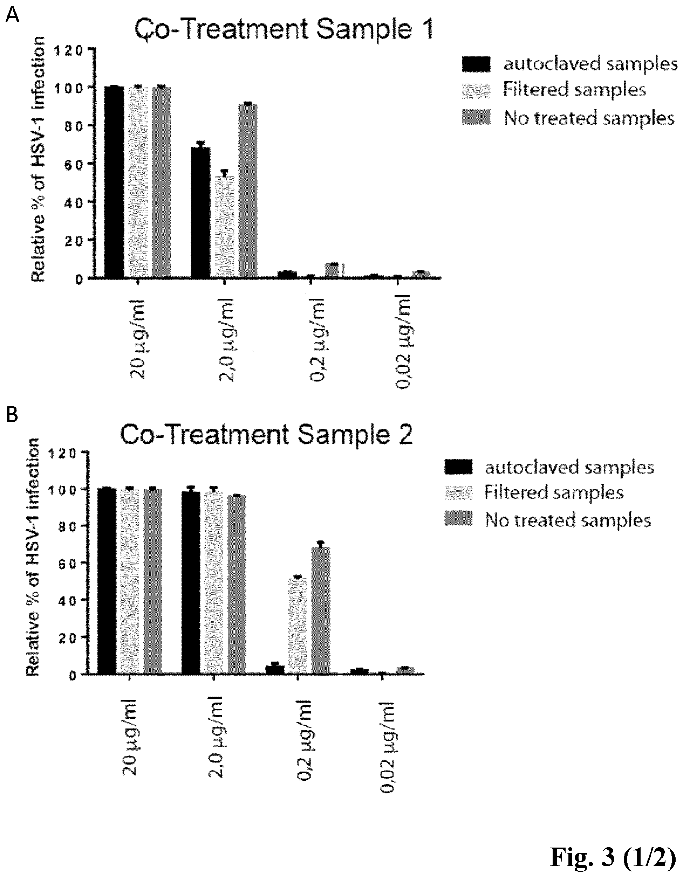

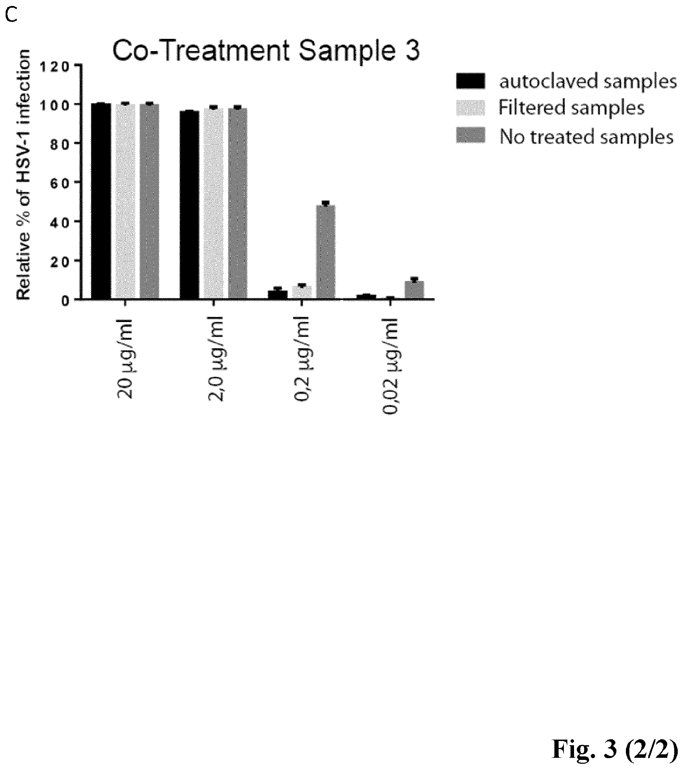

In Figure 3, CSEs of different origin and prepared by different extraction and treatment methods were compared. In particular, sample 1 (Figure 3A) refers to an extract obtained from Burned shells by Naviglio method (Naviglio extractor works at high pressure and moderate temperature); sample 2 (Figure 3B) indicates an extract obtained from Dried shells by Naviglio extraction; sample 3 (Figure 3C) refers to an extract prepared from Dried shells by boiling. As can be seen, all the three extracts show a trend in the inhibition of viral replication comparable to the reference extract. This indicates that the antiviral activity is independent not only of the type of extraction method used, but also of the origin of the extracts. Finally, even the sterilization process of the CSE, by filtration or passage in an autoclave, does not affect the antiviral power, when compared to the untreated extract.

### EXAMPLE 2

The following composition was prepared and tested on volunteers with a clinical history of injury due to HSV-1 infections of at least 5 years with at least two events / year:

| | |
|---|---|
| 1) Chestnut shells freeze-dried | 0,20% p/p |
| 2) Glycerine | 5,00% p/p |
| 3) Polyacrylamide | 1,40% p/p |
| 4) Isoparaffin | 0,70% p/p |
| 5) Laureth 7 | 0,19% p/p |
| 6) 2-phenoxyethanol | 0,10% p/p |
| 7) 3-(2-ethylhexyloxy)propane-1,2-diol | 0.10% p/p |
| 8) Purified water just enough | to100 g |

Check the pH.

Indications to 20 volunteers about the use of the preparation were the following: apply as soon as the typical tingling that indicates the appearance of the lesion is felt on the lip, apply at least 3 times a day. All the volunteers, with the exception of one, confirmed the effectiveness of the preparation in preventing the onset of the lip lesion. This efficacy was in some cases higher than that of commonly used antiherpetic drugs.

### BIBLIOGRAPHY

Abu-Lafi S, Al-Natsheh MS, Yaghmoor R, Al-Rimawi F (2017) Enrichment of Phenolic Compounds from Olive Mill Wastewater and In Vitro Evaluation of Their Antimicrobial Activities. Evid. Based Complement Alternat. Med. 2017:3706915. doi: 10.1155/2017/3706915. Arnow LE (1937) Colorimetric determination of the components of 3,4-dihydroxyphenylalaninetyrosine mixtures. J. Biol. Chem., 118:531-537.
Bontempo P, Rigano D, Doto A, Formisano C, Conte M, Nebbioso A, Carafa V, Caserta G, Sica V, Molinari AM et al, (2013) Genista sessilifolia DC. extracts induce apoptosis across a range of cancer cell lines. Cell Prolif. 46:183-192.
Bradford MM (1976) A rapid and sensitive method for the quantification of microgram quantities of protein utilizing the principle of protein dye binding. Anal. Biochem. 72:248-254.
Cavalli R, Donalisio M, Bisazza A, Civra A, Ranucci E, Ferruti P, Lembo D (2012) Enhanced antiviral activity of acyclovir loaded into nanoparticles. Methods Enzymol. 509:1-19.
Cheng HY, Lin CC, Lin TC (2002) Antiherpes simplex virus type 2 activity of casuarini from the bark of Terminalia arjuna Linn. Antiviral Res. 55:447-455.
Cuenca-Estrella M, Moore CB, Barchiesi F, Bille J, Chryssanthou E, Denning DW, Donnelly JP, Dromer F, Dupont B, Rex JH, Richardson MD, Sancak B, Verweij PE, Rodriguez-Tudela JL, AFST Subcommittee of the European Committee on Antimicrobial Susceptibility Testing (2003) Multicenter evaluation of the reproducibility of the proposed antifungal susceptibility testing method for fermentative yeasts of the Antifungal Susceptibility Testing Subcommittee of the European Committee on Antimicrobial Susceptibility Testing (AFST-EUCAST). Clin. Microbiol. Infect. 9(6):467-74. doi: 10.1046/j.1469-0691.2003.00592.x.
De Vasconcelos MC, Bennett RN, Rosa EA, Ferreira-Cardoso JV (2010) Composition of European chestnut (Castanea sativa Mill.) and association with health effects: Fresh and processed products. J. Sci. Food Agric. 90:1578-1589.
Dolatabadi S, Moghadam HN, Mahdavi-Ourtakand M (2018) Evaluating the anti-biofilm and antibacterial effects of Juglans regia L. extracts against clinical isolates of Pseudomonas aeruginosa. Microb. Pathog. 118:285-289. doi: 10.1016/j.micpath.2018.03.055.
Ferri M, Bin S, Vallini V, Fava F, Michelini E, Roda A, Minnucci G, Bucchi G, Tassoni A (2016) Recovery of polyphenols from red grape pomace and assessment of their antioxidant and anticholesterol activities. New Biotechnol. 33:338-344.
Franci G, Falanga A, Zannella C, Folliero V, Martora F, Galdiero M, Galdiero S, Morelli G, Galdiero M (2017) Infectivity inhibition by overlapping synthetic peptides derived from the gH/gL heterodimer of herpes simplex virus type 1. J. Pept. Sci. 23(4):311-319.
Franci G, Folliero V, Cammarota M, Zannella C, Sarno F, Schiraldi C, de Lera AR, Altucci L, Galdiero M (2018) Epigenetic modulator UVI5008 inhibits MRSA by interfering with bacterial gyrase. Sci. Rep. 8(1):13117. doi: 10.1038/s41598-018-31135-9.
Fuchuki K, Sakagami H, Okuda T, Hatano T, Tanuma S, Kitajima K, Inoue Y, Inoue S, Ichikawa S, et al. (1989) Inhibition of herpes simplex virus infection by tannins and related compounds. Antiviral Res. 11:285-297.
Fuwa H (1954) A new method of microdetermination of amylase activity by the use of amylase as the substrate. J. Biochem. 41:583-603.
Helfer M, Koppensteiner H, Schneider M, Rebensburg S, Forcisi S, Müller C, Schmitt-Kopplin P, Schindler M, Brack-Werne R (2014) The Root Extract of the Medicinal Plant Pelargonium sidoides Is a Potent HIV-1 Attachment Inhibitor. PLOS ONE
International Committee on Taxonomy of Viruses (2002) 61.0.6. Lentivirus. National Institutes of Health. Retrieved February 28, 2006.
International Committee on Taxonomy of Viruses (2002). 61. Retroviridae. National Institutes of Health. Retrieved February 28, 2006.
Jia Z, Tang M, Wa J (1999) The determination of flavonoid contents in mulberry and their scavenging effects on superoxide radicals. Food Chem. 64:555-559.
Jung BS, Lee NK, Na DS, Yu HH, Paik HD (2015) Comparative analysis of the antioxidant and anticancer activities of chestnut inner shell extracts prepared with various solvents. J. Sci. Food Agric. 96:2097-2102.
Lee SM, Lee YR, Cho KS, Cho YN, Lee YA, Hwang DY, Jung YJ, Son HJ (2015) Stalked sea squirt (Styela clava) tunic waste as a valuable bioresource: cosmetic and antioxidant activities. Process. Biochem. 50:1977-1984.
Lenoci A, Tomassi S, Conte MR, Benedetti R, Rodriguez V, Carradori S, Secci D, Castellano S, Sbardella G, Filetici P et al. (2014) Quinoline-Based p300 Histone Acetyltransferase Inhibitors with Pro-apoptotic Activity in Human Leukemia U937 Cells. Chem. Med. Chem. 9:542-548. doi: 10.1002/cmdc.201300536.
Levy JA (1993) HIV pathogenesis and long-term survival. AIDS. 7 (11):1401-10.
Liberti A, Goretti G, Russo MV (1983) PCDD and PCDF formation in the combustion of vegetable wastes. Chemosphere 12:661-663.
Marinova D, Ribarova F, Atanassova M (2005) Total phenolics and total flavonoids in bulgarian fruits and vegetables. J. Univ. Chem. Technol. Metallurgy 40:255-260.
Nebbioso A, Clarke N, Voltz E, Germain E, Ambrosino C, Bontempo P, Alvarez R, Schiavone EM, Ferrara F et al (2005) Tumor-selective action of HDAC inhibitors involves TRAIL induction in acute myeloid leukemia cells. Nat. Med. 11:77-84.
Nelson NA (1944) A photometric adaptation of the Somogyi method for the determination of glucose. J. Biol. Chem. 153:375-380.
Pauwels R, Balzarini J, Baba M, Snoeck R, Schols D, Herdewijn P, Desmyter J, De Clercq E (1988) Rapid and automated tetrazolium-based colorimetric assay for the detection of anti-HIV compounds. J. Virol. Methods 20(4):309-321. doi: 10.1016/0166-0934(88)90134-6.
Pendota SC, Aremu AO, Slavětínská LP, Rárová L, Grúz J, Doležal K, Van Staden J (2018) Identification and characterization of potential bioactive compounds from the leaves of *Leucosidea sericea.* J. Ethnopharmacol. 220:169-176. doi: 10.1016/j.jep.2018.03.035.
Peri C, Pompei C (1971) Estimation of different phenolic groups in vegetable extracts. Phytochemistry 10:2187-2189.
Roizman, B, Knipe DM, Whitley RJ (2007) Herpes simplex viruses. In Fields Virology eds. Fields, B.N., Knipe, D.M. and Howley, P.M., pp 2501-2601. Philadelphia: Wolters Kluwer Health/Lippincott Williams and Wilkins.
Scalbert A, Monties B, Janin G (1989) Tannins in wood: comparison of different estimation methods. J. Agric. Food. Chem. 37:1324-1329.
Singleton VL, Rossi JA Jr (1965) Colorimetry of total phenolics with phosphomolybdic-phosphotungstic acid reagents. Am. J. Enol. Viticult 16:144-158.
Squillaci G, Apone F, Sena LM, Carola A,Tito A, Bimonte M, De Lucia A, Colucci G, La Cara F, Morana A (2018) Chestnut (Castanea sativa Mill.) industrial wastes as a valued bioresource for the production of active ingredients. Proc. Biochem. 64:228-236.
Tonelli M, Vettoretti G, Tasso B, Novelli F, Boido V, Sparatore F, Busonera B, Ouhtit A, Farci P, Blois S, Giliberti G, La Colla P (2011) Acridine derivatives as anti-BVDV agents. Antiviral Res. 91(2):133-141. doi: 10.1016/j.antiviral. 2011.05.005.
Vázquez G, Fontenla E, Santos J, Freire MS, González-Álvarez J, Antorrena G (2008) Antioxidant activity and phenolic content of chestnut (Castanea sativa) shell and eucalyptus (Eucalyptus globulus). Ind. Crops Prod. 28:279-285.
Vázquez G, González-Alvarez J, Santos J, Freire MS, Antorrena G (2009) Evaluation of potential applications for chestnut (Castanea sativa) shell and eucalyptus (Eucalyptus globulus) bark extracts. Ind. Crops Prod. 29:364-370.
Vella FM, De Masi L, Calandrelli R, Morana A, Laratta B (2019) Valorization of the agro-forestry wastes from Italian chestnut cultivars for the recovery of bioactive compounds. Eur. Food Res. Technol. 245:2679-2686.
Wang J, Zhang G, Bambara RA, Li D, Liang H, Wu H, Smith HM, Lowe NR, Demeter LM, Dykes C (2011) Nonnucleoside Reverse Transcriptase Inhibitor-Resistant HIV Is Stimulated by Efavirenz during Early Stages of Infection. J. Virol. 85(20):10861-10873.
Xia S, Liu M, Wang C, Xu W, Lan Q, Feng S, Qi F, Bao L, Du L, Liu S, Qin C, Sun F, Shi Z, Zhu Y, Jiang S, Lu L (2020) Inhibition of SARS-CoV-2 (previously 2019-nCoV) infection by a highly potent pan-coronavirus fusion inhibitor targeting its spike protein that harbors a high capacity to mediate membrane fusion. Cell Res. 30(4):343-355.
Yoo YJ, Hong J, Hatch TR (1986) Comparison of alpha-amylase activities from different assay methods. Biotechnol. Bioeng. 30:147-151.
Zaim S, Chong JH, Sankaranarayanan V, Harky A (2020) COVID-19 and Multiorgan Response. Curr Probl Cardiol. 2020 Apr 28:100618. Curr Probl Cardiol. 2020 Apr 28 : 100618. doi: 10.1016/j.cpcardiol.2020.100618
Zhu N, Zhang D, Wang W, Li X, Yang B, Song J, et al. (2020) A Novel Coronavirus from Patients with Pneumonia in China, 2019". N Engl J Med. 382 (8):727-733.

## Claims

1. Extract from shells of chestnut fruits (CSE) of *Castanea sativa* Mill. and/or fractions thereof for use in the treatment and/or prevention of *Herpesviridae* and/or *Retroviridae* and/or *Coronaviridae* infections.

2. The extract and/or fractions thereof for use according to claim 1 wherein the infection is an Herpes Simplex Virus 1 or 2 (HSV-1 or HSV-2), Severe Acute Respiratory Syndrome coronavirus 2 (SARS-CoV-2) or the Human Immunodeficiency Virus (HIV) infection.

3. The extract and/or fractions thereof for use according to claim 1 or 2 wherein the chestnut shells are waste from chestnut fruits industrial peeling.

4. The extract and/or fractions thereof for use according to any one of previous claims, wherein the extract is obtained using traditional methods for the extraction of polyphenolic compounds from *Castanea sativa* (e.g. boiling water, maceration, extraction with solvents) and/or not traditional methods (e.g. microwaves, ultrasounds, supercritical extraction).

5. The extract and/or fractions thereof for use according to any one of claims 1-4 wherein the extract is obtained from industrial chestnut shells waste as burned shells *(Brulage* method), dried shells or by manual chestnut peeling.

6. A pharmaceutical composition or supplement or medical device comprising an extract from shells of chestnut fruits (CSE) of *Castanea sativa* Mill. and/or fractions thereof and at least one pharmaceutically acceptable excipient and/or carrier, for use in the treatment and/or prevention of *Herpesviridae* and/or *Retroviridae* and/or *Coronaviridae* infections.

7. A pharmaceutical composition or supplement or medical device for use according to claim 6 wherein the infection is an HSV-1, HSV-2, HIV or Sars-CoV-2 infection.

8. The pharmaceutical composition or supplement or medical device for use according to claim 6 or 7 wherein the excipient is selected from the group consisting of: emollients (e.g. glycerin and/or other), gelling (e.g. polyacrylamide, isoparaffin, laureth 7 and/or other), preservatives (e.g. 2-phenoxyethanol, 3-(2-ethylhexyloxy)propane-1,2-diol and/or other), alcohols, surfactants, bactericides and solvents (e.g. aqueous solutions, buffered physiological solution and/or other) and combinations thereof.

9. The pharmaceutical composition or supplement or medical device for use according to any one of claims 6-8 wherein the amount of the extract and/or of fractions thereof ranges from 0.000002% to 20% by weight of the total amount of the composition or supplement or device.

10. The pharmaceutical composition or supplement or medical device for use according to any one of claims 6-9 wherein the chestnut shells are waste from chestnut fruits industrial peeling.

11. The pharmaceutical composition or supplement or medical device for use according to any one of claims 6-10, wherein the extract is obtained using traditional methods for the extraction of polyphenolic compounds from *Castanea sativa* (e.g. boiling water, maceration, extraction with solvents) and/or not traditional methods (e.g. microwaves, ultrasounds, supercritical extraction).

12. The pharmaceutical composition or supplement or medical device for use according to any one of claims 6-11, wherein the extract is obtained from industrial chestnut shells waste as burned shells *(Brulage* method), dried shells or by manual chestnut peeling.

## Patentansprüche

1. Extrakt aus Schalen von Kastanienfrüchten (CSE) von *Castanea sativa* Mill und/oder Fraktionen davon zur Verwendung bei der Behandlung und/oder Vorbeugung von *Herpesviridae-* und/oder *Retroviridae-* und/oder Coronaviridae-Infektionen.

2. Der Extrakt und/oder Fraktionen davon zur Verwendung gemäß Anspruch 1, wobei die Infektion eine Herpessimplex-Virus 1- oder 2- (HSV-1 oder HSV-2), Schwere Akute Atemwegssyndrom-Coronavirus 2- (SARS-CoV-2) oder die Humane Immundefizienz-Virus- (HIV) Infektion ist.

3. Der Extrakt und/oder Fraktionen davon zur Verwendung gemäß Anspruch 1 oder 2, wobei die Kastanienschalen Abfälle aus der industriellen Schälung von Kastanienfrüchten sind.

4. Der Extrakt und/oder Fraktionen davon zur Verwendung gemäß einem der vorhergehenden Ansprüche, wobei der Extrakt unter Anwendung traditioneller Methoden für die Extraktion von polyphenolischen Verbindungen aus *Castanea sativa* (beispielsweise kochendes Wasser, Mazeration, Extraktion mit Lösungsmitteln) und/oder nicht-traditionellen Methoden (beispielsweise Mikrowellen, Ultraschall, überkritische Extraktion) gewonnen wird.

5. Der Extrakt und/oder Fraktionen davon zur Verwendung gemäß einem der Ansprüche 1-4, wobei der Extrakt aus industriellen Kastanienschalenabfällen als verbrannte Schalen (Brulage-Methode), getrocknete Schalen oder mittels manueller Kastanienschälung gewonnen wird.

6. Pharmazeutische Zusammensetzung oder Nahrungsergänzungsmittel oder Medizinprodukt, umfassend einen Extrakt aus Schalen von Kastanienfrüchten (CSE) von *Castanea sativa* **Mill** und/oder Fraktionen davon und mindestens einen pharmazeutisch annehmbaren Hilfsstoff und/oder Träger, zur Verwendung bei der Behandlung und/oder Vorbeugung von *Herpesviridae-* und/oder *Retroviridae-* und/oder Coronaviridae-Infektionen.

7. Pharmazeutische Zusammensetzung oder Nahrungsergänzungsmittel oder Medizinprodukt zur Verwendung gemäß Anspruch 6, wobei die Infektion eine HSV-1-, HSV-2-, HIV- oder SARS-CoV-2-Infektion ist.

8. Pharmazeutische Zusammensetzung oder Nahrungsergänzungsmittel oder Medizinprodukt zur Verwendung gemäß Anspruch 6 oder 7, wobei der Hilfsstoff ausgewählt ist aus der Gruppe bestehend aus Weichmachern (beispielsweise Glycerin und/oder weitere), Geliermitteln (beispielsweise Polyacrylamid, Isoparaffin, Laureth-7 und/oder weitere), Konservierungsmitteln (beispielsweise 2-Phenoxyethanol, 3-(2-Ethylhexyloxy)propan-1,2-diol und/oder weitere), Alkoholen, grenzflächenaktiven Mitteln, Bakteriziden und Lösungsmitteln (beispielsweise wässrigen Lösungen, gepufferter physiologischer Lösung und/oder weitere) und Kombinationen daraus.

9. Pharmazeutische Zusammensetzung oder Nahrungsergänzungsmittel oder Medizinprodukt zur Verwendung gemäß einem der Ansprüche 6-8, wobei die Menge des Extrakts und/oder der Fraktionen davon zwischen 0,000002 und 20 Gew.-% der Gesamtmenge der Zusammensetzung oder Ergänzung oder Produkts liegt.

10. Pharmazeutische Zusammensetzung oder Nahrungsergänzungsmittel oder Medizinprodukt zur Verwendung gemäß einem der Ansprüche 6-9, wobei die Kastanienschalen Abfälle aus der industriellen Schälung von Kastanienfrüchten sind.

11. Pharmazeutische Zusammensetzung oder Nahrungsergänzungsmittel oder Medizinprodukt zur Verwendung gemäß einem der Ansprüche 6-10, wobei der Extrakt unter Anwendung traditioneller Methoden für die Extraktion von polyphenolischen Verbindungen aus *Castanea sativa* (beispielsweise kochendes Wasser, Mazeration, Extraktion mit Lösungsmitteln) und/oder nicht-traditionellen Methoden (beispielsweise Mikrowellen, Ultraschall, überkritische Extraktion) gewonnen wird.

12. Pharmazeutische Zusammensetzung oder Nahrungsergänzungsmittel oder Medizinprodukt zur Verwendung gemäß einem der Ansprüche 6-11, wobei der Extrakt aus industriellen Kastanienschalenabfällen als verbrannte Schalen (Brulage-Methode), getrocknete Schalen oder mittels manueller Kastanienschälung gewonnen wird.

## Revendications

1. Extrait de coques de fruits de châtaignier (CSE) de *Castanea sativa* Mill et/ou de ses fractions destiné à être utilisé dans le traitement et/ou la prévention des infections par *Herpesviridae* et/ou *Retroviridae* et/ou *Coronaviridae.*

2. Extrait et/ou ses fractions destiné à être utilisé selon la revendication 1 dans lequel l'infection est une infection par virus Herpes Simplex 1 ou 2 (HSV-1 ou HSV-2), ou par le coronavirus 2 du syndrome respiratoire aigu sévère (SARS-CoV-2) ou par le virus de l'immunodéficience humaine (HIV).

3. Extrait et/ou ses fractions destiné à être utilisé selon la revendication 1 ou 2 dans lequel les coques de châtaignier sont des déchets de décollage industriel de fruits de châtaignier.

4. Extrait et/ou ses fractions destiné à être utilisé selon l'une quelconque des revendications précédentes, dans lequel l'extrait est obtenu en utilisant les procédés traditionnels pour l'extraction de composés polyphénoliques de *Castanea sativa* (par exemple l'eau bouillante, la macération, l'extraction avec des solvants) et/ou des procédés non traditionnels (par exemple les microondes, les ultrasons, l'extraction supercritique).

5. Extrait et/ou ses fractions destiné à être utilisé selon l'une quelconque des revendications 1 à 4 dans lequel l'extrait est obtenu de déchets de coques de châtaignier industriels sous forme de coques brûlées (procédé de *brûlage*)*,* de coques séchées ou par décollement de châtaignier manuel.

6. Composition pharmaceutique ou complément ou dispositif médical comprenant un extrait de coques de fruits de châtaignier (CSE) de *Castanea sativa* Mill et/ou de leurs fractions et au moins un excipient et/ou véhicule pharmaceutiquement acceptable, destiné à être utilisé dans le traitement et/ou la prévention des infections par *Herpesviridae* et/ou *Rétroviridae* et/ou *Coronaviridae.*

7. Composition pharmaceutique ou complément ou dispositif médical destiné à être utilisé selon la revendication 6 dans lequel l'infection est une infection par HSV-1, HSV-2, HIV ou Sars-CoV-2.

8. Composition pharmaceutique ou complément ou dispositif médical destiné à être utilisé selon la revendication 6 ou 7 dans lequel l'excipient est choisi dans le groupe constitué par les émollients (par exemple la glycérine et/ou autre), les agents gélifiants (par exemple polyacrylamide, isoparaffine, laureth 7 et/ou autre), les conservateurs (par exemple le 2-phénoxyéthanol, le 3-(2-éthylhexykoxy)propane-1,2-diol et/ou autre), les alcools, les tensioactifs, les bactéricides et les solvants (par exemple les solutions aqueuses, une solution physiologique tamponnée et/ou autre) et leurs combinaisons.

9. Composition pharmaceutique ou complément ou dispositif médical destiné à être utilisé selon l'une quelconque des revendications 6 à 8 dans lequel la quantité de l'extrait et/ou de ses fractions se situe dans la plage allant de 0,000002 % à 20 % en poids de la quantité totale de la composition ou du complément ou du dispositif.

10. Composition pharmaceutique ou complément ou dispositif médical destiné à être utilisé selon l'une quelconque des revendications 6 à 9 dans lequel les coques de châtaignier sont des déchets de décollage industriel de fruits de châtaignier.

11. Composition pharmaceutique ou complément ou dispositif médical destiné à être utilisé selon l'une quelconque des revendications 6 à 10, dans lequel l'extrait est obtenu en utilisant les procédés traditionnels pour l'extraction de composés polyphénoliques de *Castanea sativa* (par exemple l'eau bouillante, la macération, l'extraction avec des solvants) et/ou des procédés non traditionnels (par exemple microondes, ultrasons, extraction supercritique).

12. Composition pharmaceutique ou complément ou dispositif médical destiné à être utilisé selon l'une quelconque des revendications 6 à 11, dans lequel l'extrait est obtenu de déchets de coques de châtaignier industriels sous forme de coques brûlées (procédé du *brûlage*)*,* de coques séchées ou par décollement de châtaignier manuel.
